# NEUE EUROPÄISCHE PATENTSCHRIFT

(11) **EP 2 167 630 B2**
(45) Veröffentlichungstag und Bekanntmachung des Hinweises auf die Entscheidung über den Einspruch: **10.09.2014**
(45) Hinweis auf die Patenterteilung: 27.04.2011
(21) Anmeldenummer: 08784217.5
(22) Anmeldetag: 16.06.2008
(51) Int. Cl.: C12M 1/04, C12M 1/26, C12M 3/04, G01N 33/50

(54) **VERFAHREN ZUR UNTERSUCHUNG DER WIRKUNG EINES GASFÖRMIGEN MEDIUMS AUF EIN BIOLOGISCHES PRÜFSYSTEM UNTER VERWENDUNG EINES EXTRAZELLULÄREN METABOLISIERUNGSSYSTEMS SOWIE VORRICHTUNG ZUR DURCHFÜHRUNG DES VERFAHRENS**
METHOD FOR ANALYSING THE EFFECT OF A GASEOUS MEDIUM ON A BIOLOGICAL TEST SYSTEM USING AN EXTRACELLULAR METABOLISATION SYSTEM AND DEVICE FOR CARRYING OUT SAID METHOD
PROCÉDÉ D'ANALYSE DE L'EFFET D'UN MILIEU GAZEUX SUR UN SYSTÈME D'ESSAI BIOLOGIQUE AU MOYEN D'UN SYSTÈME DE MÉTABOLISATION EXTRACELLULAIRE ET DISPOSITIF PERMETTANT LA MISE EN OEUVRE DUDIT PROCÉDÉ

(30) Priorität: 29.06.2007 DE 102007030413
(43) Veröffentlichungstag der Anmeldung: 31.03.2010
(73) Patentinhaber: Fraunhofer-Gesellschaft zur Förderung der angewandten Forschung e.V., 80686 München (DE)
(72) Erfinder: KNEBEL, Jan, 30625 Hannover (DE); RITTER, Detlef, 30519 Hannover (DE)
(74) Vertreter: Patentanwälte Thömen & Körner
(86) Internationale Anmeldenummer: PCT/DE2008/001007
(87) Internationale Veröffentlichungsnummer: WO 2009/003441

(56) Entgegenhaltungen:
- WO-A-03/100417
- DE-A1- 19 526 533
- RITTER D ET AL: "In vitro exposure of isolated cells to native gaseous compounds Development and validation of an optimized system for human lung cells" EXPERIMENTAL AND TOXICOLOGIC PATHOLOGY, JENA, DE, Bd. 53, Nr. 5, 1. Januar 2001 (2001-01-01), Seiten 373-386, XP004956294 ISSN: 0940-2993
- AUFDERHEIDE MICHAELA ET AL: "A modified Ames assay reveals the mutagenicity of native cigarette mainstream smoke and its gas vapour phase." EXPERIMENTAL AND TOXICOLOGIC PATHOLOGY : OFFICIAL JOURNAL OF THE GESELLSCHAFT FÜR TOXIKOLOGISCHE PATHOLOGIE AUG 2007, Bd. 58, Nr. 6, 6. Juni 2007 (2007-06-06), Seiten 383-392, XP002502237 ISSN: 0940-2993

## Beschreibung

Die Erfindung betrifft ein Verfahren zur Untersuchung der Wirkung eines gasförmigen Mediums auf ein biologisches Prüfsystem unter Verwendung eines extrazellulären Metabolisierungssystems sowie Vorrichtung zur Durchführung des Verfahrens.

Es ist bekannt, zelluläre Prüfsysteme in Form von Eukaryonten-Kulturen, insbesondere Zelllinien, Primärzellen, Biopsien, Lavagen, Isolate, PCLS und dergleichen, ex-vivo Luft getragenen Substanzen nativer oder künstlicher Art auszusetzen, um die biologische Wirkung dieser Substanzen zu untersuchen. Die Eukaryonten-Kulturen werden hierbei einer künstlichen oder natürlichen Prüfatmosphäre, auch Expositionsatmosphäre genannt, ausgesetzt.

Da häufig erst die Stoffwechselzwischenprodukte der genannten Luft getragenen Substanzen biologisch aktiv sind, wird eine biologische Wirkung der Prüftmosphäre im zellulären Prüfsystem erst nachweisbar, wenn die genannten Stoffwechselzwischenprodukte im Zuge einer Metabolisierung der genannten Luft getragenen Substanzen gebildet werden konnten.

Nachfolgend sind beispielhaft zelluläre Prüfsysteme genannt, bei denen eine intrazelluläre Metabolisierungskapazität unterstellt oder nachgewiesen wurde:
(a) Isolate von Primärzellen mit recht unterschiedlich definierten und in Abhängigkeit von der jeweiligen Isolierungstechnik und dem Isolierungsbatch verschieden vorhandenen Metabolisierungsfähigkeiten/-kapazitäten (z.B. Hepatozyten der Rattenleber oder von humanen Biopsien; M.J. Gómez-Lechón et al., Hepatocytes - the choice to investigate drug metabolism and toxicity in man: In vitro variability as a reflection of in vivo, Chemico-Biol. Int. (2006), doi: 10.1016/j.cbi.2006.19.013)
(b) immortalisierte Hepatozyten-Zelllinien, die je nach Subpopulation eine unterschiedliche Metabolisierungsfähigkeit/-kapazität besitzen können (z.B. die humane Hepatomzelllinie HepG2; Aden et.al. 1979)
(c) gentechnologisch veränderte Zellsysteme (z.B. von V79 abgeleitete Zelllinien mit definierter Expression spezifischer Cytochrom P-450 Formen; A. Townsend et al., Modeling the metabloc competencay of glutathione S-transferases using genetically modified cell lines, Toxicology 181-182 (2002) 265 - 269; N. Krebsfaenger et al., V79 Chinese Hamster Cells Genetically Engineered für Polymorphic Cytochrome P450 2D6 and their Predictive Value for Humans, ALTEX 20, 3/03, 143 - 154).
(d) Kokulturen von metabolisch kompetenten Zellen und nicht metabolisierenden Zellen des primären Zielgewebes der Noxe (S. Bremer et al., Detection of the Embryotoxic Potential of Cyclophosamide by Using a Combined System of Metabolic Competent Cells and Embryonic Stem Cells, ATLA 30, 77- 85, 2002).
(e) Gewebeschnitte (D.S. Pushparajah et al., Evaluation of the precision-cut liver and lung slice systems fort he study of induction of CYP1, epoxide hydrolase and glutathione S-transferase activities, Toxicology 231 (2007) 68 - 80).

Nachteilig an diesen zellulären Prüfsystemen mit intrazellulärem Metabolisierungssystem können die Aufwendigkeit der Kulturverfahren sowie die Verfügbarkeit und Reproduzierbarkeit der Metabolisierungseffizienz sein.

Grundsätzlich erfüllen die vorgenannten zellulären Prüfsystemen mit intrazellulärem Metabolisierungssystem auch nicht zwingend die Anforderungen, die durch diverse internationale Prüfvorschriften, beispielsweise der "OECD Guideline for the Testing of Chemicals" Nr. 473, gestellt werden, welche die Gegenwart eines ausreichend effizienten Metabolisierungssystems fordern.

Um diese Prüfvorschriften zu erfüllen, können bekanntermaßen zelluläre Prüfsysteme ohne intrazelluläre Metabolisierung oder zelluläre Prüfsysteme, bei denen die Effizienz der intrazellulären Metabolisierung nicht nachgewiesen wurde, verwendet werden, die in Kulturflaschen mit ebenen Boden oder in runden Kulturflaschen, so genannten Roller-Bottles, exponiert werden. Dabei wird das zelluläre Prüfsystem mit einem Gemisch aus einem Erhaltungsmedium und einem extrazellulärem Metabolisierungssystem überschichtet und die Prüfatmosphäre kontinuierlich durch die Flasche geleitet. Zum Erreichen einer Durchmischung von Erhaltungsmedium, extrazellulärem Metabolisierungssystem und Prüfatmosphäre werden die Kulturflaschen geschüttelt, gekippt oder gedreht.

Nachteilig an diesem Verfahren ist, dass kein unmittelbarer und definierter Kontakt zwischen Prüfatmosphäre und zellulärem Prüfsystem möglich ist. Das Ergebnis ist daher mit einer geringeren Sensitivität und Spezifität sowie mit einem Fehlen einer exakten Dosimetrie behaftet.

RITTER D ET AL. ("In vitro exposure of isolated cells to native gaseous compounds Development and validation of an optimized system for human lung cells" EXPERIMENTAL AND TOXICOLOGIC PATHOLOGY, JENA, DE, Bd. 53, Nr. 5, 1. Januar 2001 (2001-01-01), Seiten 373-386) offenbart eine Variante des CULTEX Systems, welches zur Untersuchung von Gasen mittels immobilisierter Zellen Anwendung findet. Die Expositionsvorrichtung besteht aus einem Zuleitungssystem für Testgase oberhalb einer permeablen Membran auf welcher eukaryontische Testzellen als Biosensor kultiviert werden, und einem Nährmediumversorgungssystems unterhalb der Membran. Die Zellen sind nur basal in Kontakt mit dem Nährmedium, d.h. sie sind nicht vom Medium bedeckt. Die Zugabe eines Metabolisierungssystems zum Nährmedium wird nicht erwähnt.

Ausgehend von diesem Stand der Technik liegt der Erfindung die Aufgabe zugrunde, ein alternatives Verfahren mit hoher Sensitivität und Selektivität zur Untersuchung der Wirkung eines gasförmigen Mediums auf ein biologisches Prüfsystem unter Verwendung eines extrazellulären Metabolisierungssystems sowie eine Vorrichtung zur Durchführung dieses Verfahrens zur Verfügung zu stellen.

Diese Aufgabe wird durch ein Verfahren zur Untersuchung der Wirkung eines gasförmigen Mediums auf ein biologisches Prüfsystem unter Verwendung eines extrazellulären Metabolisierungssystems gemäß den Merkmalen des Anspruchs 1 sowie durch eine Expositionsvorrichtung gemäß den Merkmalen des Anspruchs 5 gelöst. Weiterbildungen und vorteilhafte Ausgestaltungen der Erfindung ergeben sich aus den Unteransprüchen.

Das erfindungsgemäße Verfahren zur Untersuchung der Wirkung eines gasförmigen Mediums auf ein biologisches Prüfsystem unter Verwendung eines extrazellulären Metabolisierungssystems, umfasst folgende Verfahrensschritte:
- Anzüchtung eines biologischen Prüfsystems auf einem permeablen Träger
- Leiten des gasförmigen Mediums über die Oberfläche des biologischen Prüfsystems zur Bildung einer Expositionsatmosphäre oberhalb des biologischen Prüfsystems
- Zugabe des extrazellulären Metabolisierungssystems zu einem Erhaltungsmedium
- Positionierung des Erhaltungsmediums mit extrazellulärem Metabolisierungssystem unterhalb des permeablen Trägers mit Kontakt zum permeablen Träger, derart, dass das Erhaltungsmedium mit extrazellulärem Metabolisierungssystem nur durch den permeablen Träger durchtritt, das biologische Prüfsystem jedoch nicht vom Erhaltungsmedium mit extrazellulärem Metabolisierungssystem überschwemmt wird.

Das erfindungsgemäße Verfahren ermöglicht mit hoher Sensitivität und Selektivität die Untersuchung der Wirkung eines gasförmigen Mediums auf ein biologisches Prüfsystem unter Verwendung eines extrazellulären Metabolisierungssystems.

Durch das erfindungsgemäße Verfahren werden die folgenden, als erfindungswesentlich erkannten, für die hohe Sensitivität und Selektivität des Verfahrens verantwortlichen und während der Exposition des biologischen Prüfsystems in der Expositionsatmosphäre gleichzeitig einzuhaltenden Bedingungen erfüllt:
a) Herstellung eines direkten und ungehinderten Kontaktes zwischen Expositionsatmosphäre und biologischem Prüfsystem
b) Herstellung eines Kontaktes zwischen der Expositionsatmosphäre und dem extrazellulären Metabolisierungssystem
c) Zugänglichmachen der Reaktionsprodukte der Metabolisierungsreaktionen des Expositionsatmosphäre mit dem extrazellulären Metabolisierungssystem für das biologische Prüfsystem
d) Zugänglichmachen des Erhaltungsmediums für das biologische Prüfsystem
e) Erhalt der Vitalität des biologischen Prüfsystems

Unabhängig davon, ob das biologische Prüfsystem intrazelluläre Metabolisierungsfähigkeiten aufweist oder ob intrazelluläre Metabolisierungsfähigkeiten im biologischen Prüfsystem vollständig fehlen, zu gering ausgeprägt, zu wenig reproduzierbar oder zu wenig spezifisch definiert sind, wird durch das erfindungsgemäße Verfahren unter Verwendung eines definierten, extrazellulären Metabolisierungssystems die Metabolisierungsfähigkeit sichergestellt.

Wichtig ist, dass quasi durch die Grenzschicht aus permeablem Träger mit darauf exponiertem biologischen Prüfsystem die Kompartimente "Expositionsatmosphäre" und "Erhaltungsmedium mit extrazellulärem Metabolisierungssystem" physikalisch voreinander getrennt sind, so dass das Erhaltungsmedium mit extrazellulärem Metabolisierungssystem in erfindungsgemäß vorgegebener Weise nur durch den permeablen Träger durchtreten kann.

Unter einem Erhaltungsmedium im Sinne der Erfindung wird hier und im Folgenden ein Kulturmedium, auch als Nährmedium bezeichnet, mit oder ohne Zusätzen oder eine Salzlösung verstanden.

Das gasförmige Medium kann im Sinne der Erfindung als reines Gas oder Gasgemisch vorliegen, d. h. alle darin enthaltenen Stoffe (Atome, Moleküle, etc.) befinden sich in der Gasphase, und/oder es kann auch als Träger für Fest- und/oder Flüssigstoffe dienen. Insbesondere können so Aerosole, zerstäubte Flüssigkeiten, kleine Flüssigkeitströpfchen (z. B. Pflanzenschutzmittel als Sprühnebel, etc.), Schwebeteilchen, Feststoffpartikel (z. B. Holzstaub, etc.), gasförmige Suspensionen, zerstäubte Suspensionen oder Emulsionen als zu tragende Substanzen in dem Trägergas enthalten sein.

Durch das erfindungsgemäße Verfahren ist es auf technisch einfache Art und in Routineanwendungen möglich, mit biologischen Prüfsystemen, die unter Umständen keine eigene Metabolisierungskapazität besitzen, sicher, d.h. unter Vitalitätserhalt, mit hoher Sensitivität des Verfahrens aufgrund des intensiven Kontakts der Expositionsatmosphäre sowohl mit dem biologischen Prüfsystem als auch mit dem extrazellulären Metabolisierungssystem und insbesondere unter Einhaltung diverser internationaler Prüfvorschriften, beispielsweise der dem Fachmann bekannten OECD 473, Untersuchungen der biologischen Wirkung von gasförmigen Medien aller Art durchzuführen.

Daneben ermöglicht das erfindungsgemäße Verfahren die Verwendung von individuell hergestellten Zusammensetzungen diverser externer, dem Fachmann bekannter Metabolisierungssysteme, beispielsweise mit Cytochromen von Labornagern oder vom Menschen, die auf die verschiedenen spezifischen Stoffwechseleigenheiten eines biologischen Prüfsystems gezielt abgestimmt sein können.

Es wird im Sinne der Erfindung vorzugsweise ein permeabler Träger verwendet, der derart ausgebildet ist, dass er einerseits zur Aufnahme des biologischen Prüfsystems geeignet ist und andererseits eine Trennung der gasförmigen Phase, also der Expositionsatmosphäre, von der flüssigen Phase, also des Erhaltungsmediums mit extrazellulärem Metabolisierungssystem, ermöglicht. Mithin kann die Porengröße und Porendichte des permeablen Trägers in Abhängigkeit vom der Art des biologischen Prüfsystems und der Art sowie Viskosität des Erhaltungsmediums variieren.

Vorzugsweise wird als permeabler Träger eine mikroporöse Membran verwendet. Vorzugsweise wird als permeabler Träger ein Gel verwendet. Zur Aufnahme des biologischen Prüfsystems kann auch ein Kulturgefäß, beispielsweise ein kommerziell erhältliches so genanntes Cell Culture Insert, vorgesehen sein, wobei der permeable Träger die Bodenmembran des Kulturgefäßes bildet.

Eine Weiterbildung der Erfindung ist gekennzeichnet durch folgenden weiteren Verfahrenschritt:
- Regulierung des Durchtritts des Erhaltungsmediums mit zugegebenem extrazellulärem Metabolisierungssystem durch den permeablen Träger durch Vorgeben eines bestimmten, auf das Erhaltungsmedium mit extrazellulärem Metabolisierungssystem einwirkenden Drucks.

Weiterhin ist vorgesehen, dass der auf das Erhaltungsmedium mit extrazellulärem Metabolisierungssystem einwirkende Druck derart gewählt wird, dass das Erhaltungsmedium mit extrazellulärem Metabolisierungssystem durch den permeablen Träger durchtritt, das biologische Prüfsystem jedoch nicht vom Erhaltungsmedium mit extrazellulärem Metabolisierungssystem überschwemmt wird.

Eine vorteilhafte Ausgestaltung der Erfindung ist durch folgenden weiteren Verfahrenschritt gekennzeichnet:
- Regulierung der Verdunstungsrate über dem biologischen Prüfsystem durch Vorgeben einer bestimmten Temperatur im unmittelbaren Umgebungsbereich des biologischen Prüfsystems und/oder Vorgeben einer bestimmten Strömungsgeschwindigkeit des über die Oberfläche des biologischen Prüfsystems strömenden gasförmigen Mediums.

Durch die Temperierung, vorzugsweise im unmittelbaren Umgebungsbereich des biologischen Prüfsystems, lässt sich die Verdunstungsrate des durch die permeable Membran durchtretenden Erhaltungsmediums mit extrazellulärem Metabolisierungssystem regulieren, um so ein Austrocknen und damit ein Absterben des biologischen Prüfsystems zu verhindern.

Die Verdunstungsrate ist auch abhängig vom Feuchtigkeitsgehalt des gasförmigen Mediums. Eine entsprechende Regulierung oder Steuerung der Strömungsgeschwindigkeit des gasförmigen Mediums über dem biologischen Prüfsystem ist erforderlich.

Die Regulierung und Steuerung dieser beiden vorgenannten Parameter sowie des auf das Erhaltungsmedium mit extrazellulärem Metabolisierungssystem einwirkenden Drucks schafft definierte Bedingungen zur Untersuchung der Wirkung eines gasförmigen Medium auf ein biologisches Prüfsystem unter Verwendung eines extrazellulärem Metabolisierungssystems.

Die Erfindung betrifft außerdem eine Expositionsvorrichtung zur Durchführung des Verfahrens nach einem der Ansprüche 1 bis 3, umfassend
- mindestens einen permeablen Träger, auf dem ein biologisches Prüfsystem angezüchtet ist,
- mindestens ein Zuleitungssystem, über das ein gasförmiges Medium über die Oberfläche des biologischen Prüfsystems zur Bildung einer Expositionsatmosphäre oberhalb des biologischen Prüfsystems leitbar ist
- ein Zuführsystem, das ein Erhaltungsmedium mit extrazellulärem Metabolisierungssystem enthält und derart ausgebildet ist, dass das Erhaltungsmedium mit extrazellulärem Metabolisierungssystem in direktem Kontakt mit der Unterseite des permeablen Trägers steht, wobei
- der permeable Träger derart ausgebildet und innerhalb der Expositionsvorrichtung angeordnet ist, dass dieser das Erhaltungsmedium mit extrazellulärem Metabolisierungssystem physikalisch von der Expositionsatmosphäre trennt, wobei
- das Erhaltungsmedium mit extrazellulärem Metabolisierungssystem mittels eines vorgegebenen Drucks definiert nur durch den permeablen Träger drückbar ist, nämlich derart, dass das Erhaltungsmedium mit extrazellulärem Metabolisierungssystem durch den permeablen Träger durchtritt, das biologische Prüfsystem jedoch nicht vom Erhaltungsmedium mit extrazellulärem Metabolisierungssystem überschwemmt wird.

Das Zuleitungssystem umfasst alle Vorrichtungen, die geeignet sind, das gasförmige Medium über die Oberfläche des biologischen Prüfsystems zur Bildung einer Expositionsatmosphäre oberhalb des biologischen Prüfsystems zu leiten. Eine derartige Vorrichtung ist beispielsweise aus der DE 100 14 057 A1 bekannt. Die Offenbarung dieser Patentanmeldung wird hiermit durch Bezugnahme vollinhaltlich in die vorliegende Anmeldung mit aufgenommen.

Das Zuleitungssystem umfasst vorzugsweise auch alle Vorrichtungen, die durch ihre Konstruktion die Heranführung des gasförmigen Mediums in besonderer Art realisieren, beispielsweise mit hyperbolischem Innenprofil zur gezielten Abscheidung von Aerosoltröpfchen.

Ferner umfasst das Zuleitungssystem vorzugsweise auch Vorrichtungen zur elektrostatischen Abscheidung von Partikeln oder Tröpfchen und/oder eine Aufladungsvorrichtung. Derartige Vorrichtungen sind beispielsweise aus der DE 195 26 533 A1 bekannt. Die Offenbarung dieser Patentanmeldung wird hiermit ebenfalls durch Bezugnahme vollinhaltlich in die vorliegende Anmeldung mit aufgenommen.

Einer Weiterbildung der Erfindung sieht vor, dass das Zuführsystem gegenüber dem Zuleitungssystem und der Expositionsatmosphäre abgedichtet ist und lediglich durch den permeablen Träger hindurch das Erhaltungsmedium samt extrazellulärem Metabolisierungssystem mit der Expositionsatmosphäre in Kontakt treten kann.

Der vorgegebene Druck ist hydrostatisch oder mittels wenigstens einer Pumpe oder anderer Druck erzeugender Mittel einstellbar.

Gemäß einer Weiterbildung der Erfindung ist das Zuführsystem derart ausgebildet ist, dass das darin enthaltene Erhaltungsmedium mit extrazellulärem Metabolisierungssystem für die Dauer der Untersuchung im Zuführsystem verbleibt.

Vorzugsweise ist der permeable Träger derart ausgebildet, dass er einerseits die Aufnahme des biologischen Prüfsystems und andererseits eine Trennung der gasförmigen Phase, also der Expositionsatmosphäre, von der flüssigen Phase, also des Erhaltungsmediums mit extrazellulärem Metabolisierungssystem, ermöglicht. Letzteres ist insbesondere abhängig von der Art des biologischen Prüfsystems sowie der Art und Viskosität des Erhaltungsmediums mit extrazellulärem Metabolisierungssystem.

Eine vorteilhafte Ausgestaltung der Erfindung sieht vor, dass ein Ableitungssystem vorgesehen ist, über das die Expositionsatmosphäre nach einer vorgegebenen Verweilzeit innerhalb der Expositionsvorrichtung aus dieser ableitbar ist.

Weiterhin ist vorgesehen, dass das Zuleitungs- und/oder Ableitungssystem derart ausgebildet ist, dass das gasförmige Medium kontrolliert mittels eines Unterdruck-Systems oder eines Überdruck-Systems durch die Expositionsvorrichtung leitbar ist.

Bevorzugtes Mittel zum kontinuierlichen Erzeugen einer entsprechenden Druckdifferenz zwischen Zuleitungs- und/oder Ableitungssystem ist eine Pumpe, die mit strömungstechnisch im Ableitungssystem angeordnet ist. Zum vorteilhaften Einstellen der Strömungsgeschwindigkeit des Mediums ist die Pumpe in ihrer Pumpleistung dabei besonders bevorzugt steuerbar.

Zweckmäßigerweise ist gemäß einer Weiterbildung der Erfindung ein Abführsystem vorgesehen, über das das Erhaltungsmedium mit Metabolisierungssystem nach einer vorgegebenen Verweilzeit innerhalb der Expositionsvorrichtung aus dieser abführbar ist.

Vorzugsweise ist das Zuführ- und Abführsystem derart ausgebildet, dass das darin enthaltene Erhaltungsmedium mit Metabolisierungssystem in einem kontinuierlichen oder pulsierenden Fluss durch das Zuführ- und Abführsystem geführt wird, wobei zu diesem Zweck vorzugsweise wenigstens eine Pumpe vorgesehen ist.

Gemäß einer vorteilhaften Ausgestaltung der Erfindung ist wenigstens eine Heizvorrichtung vorgesehen, durch die die Expositionsvorrichtung ganz oder teilweise temperierbar ist.

Durch die Temperierung, vorzugsweise im unmittelbaren Umgebungsbereich de des biologischen Prüfsystems, lässt sich die Verdunstungsrate des durch die permeable Membran durchtretenden Erhaltungsmediums mit extrazellulärem Metabolisierungssystem regulieren, um so ein Austrocknen und damit ein Absterben des biologischen Prüfsystems zu verhindern.

Die Verdunstungsrate ist auch abhängig vom Feuchtigkeitsgehalt des gasförmigen Mediums. Eine entsprechende Regulierung oder Steuerung der Strömungsgeschwindigkeit des gasförmigen Mediums über dem biologischen Prüfsystem ist erforderlich.

Gemäß einer vorteilhaften Ausgestaltung der Erfindung umfasst die erfindungsgemäße Expositionsvorrichtung entsprechend auch eine Vorrichtung zur Regulierung und Steuerung der Strömungsgeschwindigkeit des gasförmigen Mediums über dem biologischen Prüfsystem.

Die Regulierung und Steuerung dieser beiden vorgenannten Parameter sowie des erforderlichen auf das Erhaltungsmedium mit extrazellulärem Metabolisierungssystem einwirkenden Drucks schafft definierte Bedingungen zur Untersuchung der Wirkung eines gasförmigen Medium auf ein biologisches Prüfsystem unter Verwendung eines extrazellulärem Metabolisierungssystems.

Vorzugsweise ist das verwendete biologische Prüfsystem entweder eine Eukaryonten-Kultur, besonders bevorzugt Zelllinien, Primärzellisolate, Gewebeschnitte, rekonstruierte Gewebe wie Kokulturen, oder genetisch veränderte Zellen, oder eine Prokaryonten-Kultur. Vorzugsweise wird ein solches biologisches Prüfsystems bei dem zuvor genannten erfindungsgemäßen Verfahren verwendet.

Eine Weiterbildung der Erfindung sieht vor, dass die Expositionsvorrichtung mit einer zellbasierten Sensorik kombiniert ist.

Unter dem Begriff "zellbasierter Sensorik" werden alle Vorrichtungen verstanden, die geeignet sind, den biologischen Status der eingesetzten biologischen Prüfsysteme während ihrer Positionierung auf dem permeablen Träger in der Expositionsvorrichtung zu untersuchen.

Insbesondere kann dies in Form einer Online-Messung realisiert sein oder einer Analytik, die geeignet ist, innerhalb von Intervallen kürzeren oder längeren zeitlichen Abstandes einen Messwert auszulesen.

Zellbasierte Sensorik kann z.B. für Endpunkte realisiert werden, die auf dem Einsatz einer Fluoreszenz- oder Lumineszenzmessung basieren. Dazu wird das biologische Prüfsystem z.B. mit einem Fluorophor ausgestattet, dessen Eigenschaften in Abhängigkeit vom zellulären Zustand stehen und diesen daher über die Fluoreszenzanalytik unter bestimmten Aspekten abbilden können, beispielsweise durch Anfärbung der Zellen mit H₂DCFDA zum Nachweis intrazellulärer Radikalbildung. Mittels einer geeigneten Optik, beispielsweise einer Lichtleitertechnik, und externer Lichtquelle und Detektor sowie Steuereinrichtung und Messwertaufnahme kann dann direkt am biologischen Prüfsystem auf der Membran die Lichtanregung, also Excitation, und Emissionsmessung, also Emission, stattfinden. Eine entsprechende Einrichtung kann z.B. auch für die Analyse von Lumineszenzerscheinungen z.B. im Zusammenhang mit Expressionsanalysen, beispielsweise Reportergen-Assays, vorgesehen sein.

Zellbasierte Sensorik kann beispielsweise auch durch elektrische oder elektrochemische Messungen realisiert werden, insbesondere in Form einer elektrischen Widerstandmessung, also TEER, trans epithelial electrical resistance, oder in Form einer Impedanz-Messung.

Eine weitere Ausführung zellbasierter Sensorik kann auch eine Einrichtung sein, die geeignet ist, von der Zelle abgegebene Substanzen, beispielsweise Enzyme wie Lactat-Dehydrogenase oder Zytokine, im Erhaltungsmedium quantitativ oder qualitativ zu analysieren.

Insbesondere sind diese Einrichtungen geeignet, eine Analytik während eines Expositionsvorganges zuzulassen.

Die Anmeldung betrifft ferner die Verwendung des Verfahrens nach einem der Ansprüche 1 bis 3 oder der Expositionsvorrichtung nach einem der Ansprüche 4 bis 15 zur Exposition wenigstens eines biologischen Prüfsystems in Zigarettenrauch oder dergleichen oder in Abgasen, vorzugsweise in Automobilabgasen oder in Industrieabgasen.

Die Erfindung betrifft außerdem die Verwendung des Verfahrens nach einem der Ansprüche 1 bis 3 oder der Expositionsvorrichtung nach einem der Ansprüche 4 bis 15 zur Untersuchung von Umweltatmosphären, Arbeitsplatzatmosphären oder Innenraumatmosphären.

Die Anmeldung betrifft weiterhin die Verwendung des Verfahrens nach einem der Ansprüche 1 bis 3 oder der Expositionsvorrichtung nach einem der Ansprüche 4 bis 15 zur Untersuchung der Wirkung eines gasförmigen Mediums auf ein biologisches Prüfsystem auf dem Gebiet Produktsicherheit, Verbraucherschutz, Pharmaentwicklung, Produktionsüberwachung oder Medizintechnik.

Die Anmeldung betrifft ferner die Verwendung des Verfahrens nach einem der Ansprüche 1 bis 3 oder der Expositionsvorrichtung nach einem der Ansprüche 4 bis 15 zur Untersuchung der Wirkung eines gasförmigen Mediums auf ein biologisches Prüfsystem unter Einhaltung regulatorischer Richtlinien, vorzugsweise von OECD-Richtlinien.

Eine bevorzugte Verwendung des Verfahrens nach einem der Ansprüche 1 bis 3 oder der Expositionsvorrichtung nach einem der Ansprüche 4 bis 15 ist die zulassungsrelevante Prüfung von technisch hergestellten Atmosphären, also beispielsweise Gaszubereitungen oder -mischungen, die für ihre Herstellung und den Vertrieb nach Chemikalienrecht eine behördliche Zulassung erfordern und deren Unbedenklichkeitsnachweise nach EU-Recht möglichst unter Verwendung von tierversuchsfreier Prüfungen durchzuführen sind.

Die Anmeldung betrifft ferner die Verwendung des Verfahrens nach einem der Ansprüche 1 bis 3 oder der Expositionsvorrichtung nach einem der Ansprüche 4 bis 15 zur Untersuchung der Wirkung eines gasförmigen Mediums auf ein biologisches Prüfsystem, nämlich zur Untersuchung toxikologischer Effekte, gentoxischer Effekte, immunmodulatorischer oder immuntoxischer Effekte oder anderer biologisch oder toxikologisch relevanter zellulärer Veränderungen.

Nachfolgend wird die Erfindung anhand eines Ausführungsbeispiels erläutert, dass in der Zeichnung dargestellt ist. In dieser zeigen
- Fig. 1: schematisch eine Teilansicht einer zur Durchführung des erfindungsgemäßen Verfahrens geeigneten Expositionsvorrichtung und
- Fig. 2: einen schematischen Aufbau für eine mögliche Untersuchung der Wirkung von n-Butan auf ein biologisches Prüfsystem unter Verwendung eines extrazellulären Metabolisierungssystems.

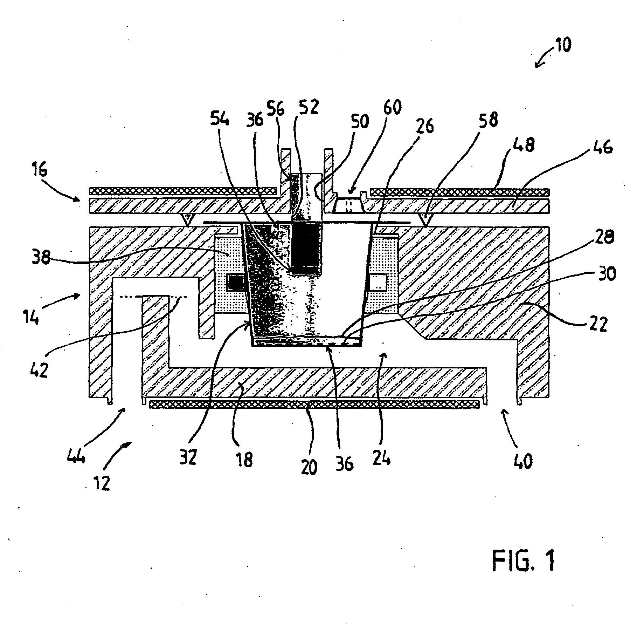

Die in Fig. 1 schematisch in Teilansicht dargestellte Expositionsvorrichtung 10, die für Feldversuche auch transportierbar ist, weist eine Unterkonstruktion 12, eine auf der Unterkonstruktion 12 angeordnete Mittelkonstruktion 14 und eine auf der Mittelkonstruktion 14 angeordnete Oberkonstruktion 16 auf.

Die Unterkonstruktion 12 ist in Form einer Wanne 18 ausgebildet, die vorzugsweise aus einem Polycarbonat besteht. Unterhalb der Wanne 18 ist eine elektrische Heizvorrichtung 20 angeordnet, die eine Temperierung der Expositionsvorrichtung 10 ermöglicht.

Weiterhin sind ein hier nicht dargestellter Vorratsbehälter zum Aufnehmen eines Erhaltungsmediums mit erfindungsgemäß zugesetztem Metabolisierungssystem und eine hier nicht dargestellte Schlauchpumpe zum Fördern des Erhaltungsmediums mit Metabolisierungssystem vom Vorratsbehälter in die Wanne 18 vorgesehen.

Die Wanne 18 der Unterkonstruktion 12 ist mit einer Platte 22 der Mittelkonstruktion 14 unter Ausbildung wenigstens eines Hohlraums 24, dem das Erhaltungsmedium mit Metabolisierungssystem zuführbar ist, dicht verbunden. Die Platte 22 besteht vorzugsweise aus einem Polycarbonat.

Es wird darauf hingewiesen, dass die Begriffe Wanne 18 und Platte 22 nicht so zu verstehen sind, dass die Wanne 18 immer einen Boden mit nach oben gezogenen Wänden aufweist und die Platte 22 nur flach ausgebildet ist. Im Grunde kann die Wanne 18 auch flach ausgebildet sein und die Platte 22 nach unten gezogene Wände aufweisen. Dies ist so beispielsweise in Fig. 1 dargestellt. Auch diverse Übergangsstadien zwischen Wanne 18 und Platte 22 mit diversen Profilierungen sind möglich.

Die Platte 18 weist weiterhin wenigstens ein Aufnahmemittel, vorliegend in Form eines Loches 26, für die Aufnahme eines mit einem biologischen Prüfsystem 28 versehenen, permeablen Trägers 30, vorliegend in Form eines Kulturgefäßes 32 auf.

Das in Fig. 1 dargestellte Kulturgefäß 32 hat eine becherartige Form mit kreisförmigem Querschnitt, wobei sich der Durchmesser von der Becheröffnung 34 bis zum Becherboden 36 konisch verjüngt. Der Becherboden 36 besteht aus einem porösen Kunststoffmaterial, zum Beispiel aus Polyethylenterephthalat. Das Kulturgefäß 32 stellt eine flüssigkeitsdurchlässige Tragstruktur für den permeablen Träger 30, insbesondere einer mikroporösen Membran 30 dar, die je nach Erfordernis der zu kultivierenden Zellen aus unterschiedlichen Kunststoffmaterialien hergestellt sein kann, z. B. ebenfalls Polyethylenterephthalat. Die mikroporöse Membran 30 trägt dabei das biologische Prüfsystem 28.

Jedes Kulturgefäß 32 ragt mit seinem Becherboden 36 in den durch Wanne 18 und Platte 22 gebildeten Hohlraum 24. Die Becheröffnung 34 befindet sich oberhalb der Platte 22.

Wichtig ist, dass das in jedem Loch 26 der Platte 18 aufgenommene Kulturgefäß 32 an der äußeren Becherwand gegenüber der Platte 18 mittels einer Dichtung 38, vorzugsweise mittels einer Silikonwulst, abgedichtet ist. Dies ist erfindungswesentlich, weil nur so sichergestellt werden kann, dass das Erhaltungsmedium mit Metabolisierungssystem nur durch die mikroporöse Membran 30 durchtreten und mit der Expositionsatmosphäre in Kontakt treten kann. Dadurch wird mithin verhindert, dass das Erhaltungsmedium mit Metabolisierungssystem am Kulturgefäß 32 vorbei nach oben gedrückt wird und dann nachteilig von oben in die Becheröffnung 34 und mithin in das Kulturgefäß 32 gelangt oder die Platte 18 überschwemmt.

Vorzugsweise erfolgt die Zufuhr des Erhaltungsmediums mit Metabolisierungssystem über eine Einlassöffnung 40 im Boden der Wanne 18. Das Erhaltungsmedium mit Metabolisierungssystem füllt dann den Hohlraum 24 zwischen Wanne 18 und Platte 22 und kommt an der Unterseite der mikroporösen Membran 30 in Kontakt mit dieser 30. Um nun das Metabolisierungssystem im Erhaltungsmedium durch die mikroporöse Membran 30 zu dem auf der Membran 30 angezüchteten biologischen Prüfsystem 28 drücken zu können, muss Druck auf das Erhaltungsmedium mit Metabolisierungssystem ausgeübt werden. Dies geschieht im einfachsten Fall hydrostatisch. Hierzu wird das Erhaltungsmedium mit Metabolisierungssystem mittels der Schlauchpumpe auf ein Niveau 40 innerhalb des Hohlraums 24 gepumpt, das oberhalb des Becherbodens 36, also oberhalb der mikroporösen Membran 30, liegt. Durch vorzugsweises Verschieben des Kulturgefäßes 32, d.h. durch Verändern des Niveaus des permeablen Trägers 30, kann der Druck ebenfalls verändert werden.

Das erforderliche Niveau bzw. der erforderliche Druck, der notwendig ist, um das Metabolisierungssystem mit Erhaltungsmedium erfindungsgemäß durch die mirkroporöse Membran 30 zu drücken, ist insbesondere abhängig von der Art der mikroporösen Membran 30, also von der Porengröße und Porendichte, und des verwendeten Erhaltungsmediums mit extrazellulärem Metabolisierungssystem. Der erforderliche Druck wird mithin empirisch ermittelt.

Vorzugsweise weist die Expositionsvorrichtung 10 im Boden der Wanne 18 eine Auslassöffnung 44 auf, über die verbrauchtes Erhaltungsmedium mit extrazellulärem Metabolisierungssystem aus der Expositionsvorrichtung 10 gelassen werden kann, vorliegend dadurch, dass das Erhaltungsmedium mit Metabolisierungssystem das genannte Niveau 40 übersteigt.

Auf der Platte 22 der Mittelkonstruktion 14 ist eine Oberkonstruktion 16 angeordnet, die einen Deckel 46 mit heruntergezogen Wänden umfasst.

Auf dem Deckel 46 kann eine weitere Heizvorrichtung 48 für die Temperierung der Expositionsvorrichtung 10 vorgesehen sein.

Innerhalb des Deckels 46 ist wenigstens ein Loch 50 vorgesehen, das über einem in der Platte 22 aufgenommenen Kulturgefäß 32 positioniert ist.

In diesem Loch 50 ist ein Strömungseinleitrohr 52 für das gasförmige Medium angeordnet, welches mit einem Ende 54 direkt in das Kulturgefäß 32 ragt, wobei das Ende 54 kurz oberhalb des biologischen Prüfsystems 28 positioniert ist. Das andere Ende 56 des Strömungseinleitrohrs 52 ist außerhalb des Deckels 46 positioniert. Das Strömungseinleitrohr 52 ist außen gegenüber dem Deckel 46 abgedichtet.

Zwischen Platte 22 und Deckel 46 ist um jedes Kulturgefäß 18 eine weitere Dichtung, vorzugsweise in Form eines Dichtrings 58, angeordnet.

Innerhalb des Dichtrings 58 ist im Deckel 46 noch in unmittelbarer Nähe des vorgenannten Lochs 50 eine Austrittsöffnung 60 für das gasförmige Medium angeordnet.

Die Austrittsöffnung 60 ist vorzugsweise mit einer hier nicht dargestellten Vakuumpumpe verbunden, um ein gasförmiges Medium durch das Strömungseinleitrohr 52 auf die Oberfläche des biologischen Prüfsystems 28 und anschließend durch die Austrittsöffnung 60 zu saugen.

Das gasförmige Medium kann bei Anwendung der Expositionsvorrichtung 10 in einem Feldversuch aus der Außenatmosphäre kommen. Somit können beispielsweise die Wirkungen verschiedener natürlich vorkommender Atmosphären auf ein biologisches Prüfsystem 28 untersucht werden.

Es ist natürlich auch möglich, dass zu untersuchende gasförmige Medium durch das Strömungseinleitrohr 52 ohne Vakuumpumpe strömen zu lassen. Dazu ist das Strömungsleitrohr 55 mit einer hier nicht dargestellten unter Druck stehenden Vorratsflasche verbunden.

Wichtig ist allein, dass zwischen Strömungseinleitrohr 52 und Austrittsöffnung 60 eine Druckdifferenz besteht, so dass das gasförmige Medium kontinuierlich über die Oberfläche des biologischen Prüfsystems 28 und erfindungsgemäß über das Metabolisierungssystem strömen kann.

Die Schlauchpumpe wird über eine hier nicht dargestellte Steuer-/Regelungseinheit bedient, um das biologische Prüfsystem 28 im Kulturgefäß 32 einerseits mit dem Erhaltungsmedium zu versorgen, und erfindungsgemäß andererseits, um den Durchtritt des dem Erhaltungsmedium zugegebenen Metabolisierungssystems durch den permeablen Träger 30 zu ermöglichen, nämlich derart, dass das Erhaltungsmedium mit Metabolisierungssystem mittels der Schlauchpumpe auf ein Niveau 40 innerhalb des Hohlraums 24 gepumpt wird, das oberhalb des Becherbodens 36, also oberhalb der mikroporösen Membran 30, liegt. Der auf das Erhaltungsmedium mit Metabolisierungssystem hydrostatisch einwirkende Druck drückt das Metabolisierungssystem nur soweit durch den permeablen Träger 30, dass das biologische Prüfsystem 28 nicht vom Erhaltungsmedium mit Metabolisierungssystem überschwemmt wird.

Die Schlauchpumpe ist also nicht nur dazu da, Erhaltungsmedium in den Hohlraum 24 zur Versorgung des biologischen Prüfsystems 28 zu pumpen, sondern sorgt auch dafür, dass das Erhaltungsmedium mit Metabolisierungssystem im Hohlraum 24 zwischen Wanne 18 und Platte 22 derart mit Druck beaufschlagt wird, dass das Metabolisierungssystem durch die vorliegend mikroporöse Membran30 gedrückt werden kann, ohne dass das Erhaltungsmedium mit Metabolisierungssystem das biologische Prüfsystem 28 überschwemmt. Eine Submersion des biologischen Prüfsystems 28 ist hier erfindungsgemäß gänzlich unerwünscht.

Um auf zuverlässige Weise zu erreichen, dass das Metabolisierungssystem in unmittelbare Nähe der oder in Kontakt mit dem biologischen Prüfsystem 28 kommt, dieses aber nicht vom Erhaltungsmedium mit Metabolisierungssystem überschwemmt wird, kann es sinnvoll sein, wenigstens einen hier nicht dargestellten Sensor zur Bestimmung des Durchtritts des Erhaltungsmediums mit Metabolisierungssystem durch die mikroporöse Membran 30 zu verwenden, welcher mit der Steuer-/Regeleinheit der Schlauchpumpe verbunden ist und entsprechende Signale an die Steuer-/Regeleinheit zum An-/Ausschalten der Schlauchpumpe gibt.

Es können ferner hier nicht dargestellte Temperatursensoren am oder innerhalb des mit Erhaltungsmedium und Metabolisierungssystem gefüllten Hohlraums 24 vorgesehen sein, und zwar für eine Temperaturregelung des Erhaltungsmediums mit Metabolisierungssystem mittels der zuvor genannten Heizvorrichtungen 20, 48.

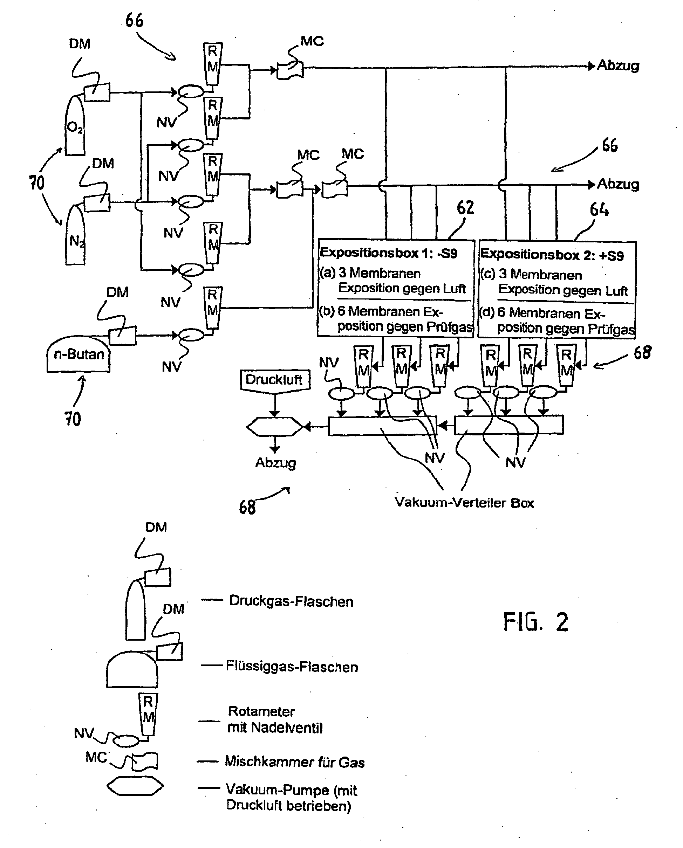

Fig. 2 zeigt einen schematischen Aufbau für eine mögliche Untersuchung der Wirkung von n-Butan auf ein biologisches Prüfsystem unter Verwendung eines extrazellulären Metabolisierungssystems.

Für die Untersuchung werden zwei Expositionsvorrichtungen 62 und 64, vorliegend als Expositionsbox 1 und Expositionsbox 2 bezeichnet, verwendet.

Jede Expositionsvorrichtung 62, 64 weist neun permeable Träger in Form von mikroporösen Membranen auf. Als biologisches Prüfsystem werden Lungenzellen der Linie V-79 verwendet. Diese wurden zuvor biphasisch, d.h. an der Luft- Flüssigkeitsgrenzschicht, auf den mikroporösen Membranen kultiviert und anschließend in die jeweilige Expositionsvorrichtung 62, 64, so wie in Fig. 1 dargestellt, eingebracht.

Jede Expositionsvorrichtung 62, 64 ist mit einem Erhaltungsmedium, nämlich mit einem Kulturmedium befüllt, wobei nur dem Kulturmedium der Expositionsvorrichtung 64 ein extrazelluläres Metabolisierungssystem, nämlich ein 3% S9-Mix zugesetzt worden ist.

Ein S9-Mix ist dem Fachmann bekannt, wird aber beispielhaft auch bei "D. M. Maron und B. N. Ames, Revised Methods for the Salmonella mutagenicity test, Mutation Research, 113 (1983) 173 - 215" beschrieben.

Sowohl das Erhaltungsmedium ohne extrazellulärem Metabolisierungssystem, also ohne S9-Mix, als auch das Erhaltungsmedium mit extrazellulärem Metabolisierungssystem, also mit S9-Mix, stehen gemäß dem erfindungsgemäßen Aufbau mit jeder mikroporösen Membran basal in Kontakt, und werden jeweils durch diese von der Expositionsatmosphäre, die von apikal die mikroporöse Membran erreicht, getrennt.

Das Erhaltungsmedium ohne oder mit extrazellulärem Metabolisierungssystem wird kontinuierlich und reproduzierbar temperiert und wirkt mit einem geeigneten vorgegebenen Druck von unten auf die mikroporösen Membranen in Abhängigkeit von der vorhandenen/nicht vorhandenen S9-Komponente im Erhaltungsmedium.

In diesen Expositionsvorrichtungen 62, 64 werden die auf den mikroporösen Membranen befindlichen biologischen Prüfsystemen aus dem Zellkulturlabor zu einem laborfernen Expositionsort transportiert.

Dort werden die Expositionsvorrichtungen 62, 64 mit den Zuleitungs- 66 und Ableitungssytemen 68 der Prüf- 70 und Referenzatmosphäre 72 verbunden.

Die Prüfatmosphäre n-Butan 70 ist mit Reinluft oder einem Gemisch aus Stickstoff und Sauerstoff verdünnt. In der Endkonzentration befinden sind 20,5 % Sauerstoff.

Es erfolgt parallel und gleichzeitig eine Exposition gegen eine Reinluft-Kontrolle sowie eine gleichzeitige Exposition von biologischen Prüfsystemen mit und ohne Zumischung von S9-Mix im Kulturmedium.

Die Zuleitung der Prüf- und Referenzatmosphäre 70, 72 zu den biologischen Prüfsystemen wird mit einer Flussrate von 10 ml/min/cm² durch Regelung der Flussrate in einem Unterdruck-System konstant über den Expositionszeitraum gewährleistet. Die Prüf- bzw. Referenzatmosphäre 70, 72 wird über den biologischen Prüfsystem, also den Zellrasen, geführt.

Die Expositionszeit beträgt mindestens 3 Stunden.

Anschließend werden die Expositionsvorrichtungen 62, 64 von ihren Zuleitungs-66 und Ableitungssystemen 68 getrennt und in das Zellkulturlabor zurücktransportiert.

Die mikroporösen Membranen mit ihren biologischen Prüfsystemen werden entnommen.

Es schließt sich die Aufarbeitung der biologischen Prüfsysteme, also der V79-Zellen, an: Analyse der Toxizität mittels Neutralrot-Assay, Untersuchung der Laktat-Dehydrogenase-Freisetzung, Analyse von Apoptose mittels Annexin-V-Assay, Untersuchung von oxidativem Stress mittels Analyse des intrazellulären Glutathion-Status, Untersuchung der Gentoxizität mittels Mikronukleus- und COMET-Assay.

### Bezugszeichenliste

(ist Teil der Beschreibung)
- 10: Expositionsvorrichtung
- 12: Unterkonstruktion
- 14: Mittelkonstruktion
- 16: Oberkonstruktion
- 18: Wanne
- 20: Heizvorrichtung
- 22: Platte
- 24: Hohlraum
- 26: Loch
- 28: biologisches Prüfsystem
- 30: permeabler Träger
- 32: Kulturgefäß
- 34: Becheröffnung
- 36: Becherboden
- 38: Dichtung
- 40: Einlassöffnung
- 42: Niveau
- 44: Auslassöffnung
- 46: Deckel
- 48: Heizvorrichtung
- 50: Loch
- 52: Strömungseinleitrohr
- 54: Ende
- 56: Ende
- 58: Dichtring
- 60: Austrittsöffnung
- 62: Expositionsvorrichtung
- 64: Expositionsvorrichtung
- 66: Zuleitungssystem
- 68: Ableitungssystem
- 70: Prüfatmosphäre
- 72: Referenzatmosphäre

## Patentansprüche

1. Verfahren zur Untersuchung der Wirkung eines gasförmigen Mediums auf ein biologisches Prüfsystem (28) unter Verwendung eines extrazellulären Metabolisierungssystems, umfassend folgende Verfahrensschritte
- Anzüchtung eines biologischen Prüfsystems (28) auf einem permeablen Träger (30)
- Leiten des gasförmigen Mediums über die Oberfläche des biologischen Prüfsystems (28) zur Bildung einer Expositionsatmosphäre oberhalb des biologischen Prüfsystems (28)
- Zugabe des extrazellulären Metabolisierungssystems zu einem Erhaltungsmedium
- Positionierung des Erhaltungsmediums mit extrazellulärem Metabolisierungssystem unterhalb des permeablen Trägers (30) mit Kontakt zum permeablen Träger (30), derart, dass das Erhaltungsmedium mit extrazellulärem Metabolisierungssystem nur durch den permeablen Träger (30) durchtritt, das biologische Prüfsystem (28) jedoch nicht vom Erhaltungsmedium mit extrazellulärem Metabolisierungssystem überschwemmt wird.

2. Verfahren nach Anspruch 1, **gekennzeichnet durch** folgenden weiteren Verfahrenschritt:
- Regulierung des Durchtritts des Erhaltungsmedium mit zugegebenem extrazellulären Metabolisierungssystems **durch** den permeablen Träger (30) **durch** Vorgeben eines bestimmten, auf das Erhaltungsmedium mit extrazellulärem Metabolisierungssystem einwirkenden Drucks.

3. Verfahren nach einem der Ansprüche 1 bis 2, **gekennzeichnet durch** folgenden weiteren Verfahrenschritt:
- Regulierung der Verdunstungsrate über der Kultur (28) **durch** Vorgeben einer bestimmten Temperatur im unmittelbaren Umgebungsbereich des biologischen Prüfsystems (28) und/oder Vorgeben einer bestimmten Strömungsgeschwindigkeit des über die Oberfläche des biologischen Prüfsystems (28) strömenden gasförmigen Mediums.

4. Expositionsvorrichtung (10) zur Durchführung des Verfahrens nach einem der Ansprüche 1 bis 3, umfassend
- mindestens einen permeablen Träger (30), auf dem ein biologisches Prüfsystem (28) angezüchtet ist,
- mindestens ein Zuleitungssystem (52), über das ein gasförmiges Medium über die Oberfläche des biologischen Prüfsystems (28) zur Bildung einer Expositionsatmosphäre oberhalb des biologischen Prüfsystems (28) leitbar ist
- ein Zuführsystem (24, 40), das ein Erhaltungsmedium mit extrazellulärem Metabolisierungssystem enthält und derart ausgebildet ist, dass das Erhaltungsmedium mit extrazellulärem Metabolisierungssystem in direktem Kontakt mit der Unterseite des permeablen Trägers (30) steht, wobei
- der permeable Träger (30) derart ausgebildet und innerhalb der Expositionsvorrichtung (10) angeordnet ist, dass dieser (30) das Erhaltungsmedium mit extrazellulärem Metabolisierungssystem physikalisch von der Expositionsatmosphäre trennt, wobei
- das Erhaltungsmedium mit extrazellulärem Metabolisierungssystem mittels eines vorgegebenen Drucks definiert nur durch den permeablen Träger (30) drückbar ist, nämlich derart, dass das Erhaltungsmedium mit extrazellulärem Metabolisierungssystem durch den permeablen Träger (30) durchtritt, das biologische Prüfsystem (28) jedoch nicht vom Erhaltungsmedium mit extrazellulärem Metabolisierungssystem überschwemmt wird.

5. Expositionsvorrichtung (10) nach Anspruch 4, **dadurch gekennzeichnet, dass** das Zuführsystem (24, 40) gegenüber dem Zuleitungssystem (50, 52) und der Expositionsatmosphäre abgedichtet ist und lediglich durch den permeablen Träger (30) hindurch das Erhaltungsmedium samt extrazellulärem Metabolisierungssystem mit der Expositionsatmosphäre unter vorgegebenen Bedingungen in Kontakt treten kann.

6. Expositionsvorrichtung (10) nach Anspruch 4, **dadurch gekennzeichnet, dass** der vorgegebene Druck hydrostatisch oder mittels wenigstens einer Pumpe einstellbar ist.

7. Expositionsvorrichtung (10) nach einem der Ansprüche 4 bis 6, **dadurch gekennzeichnet, dass** das Zuführsystem (24, 40) derart ausgebildet ist, dass das darin enthaltene Erhaltungsmedium mit extrazellulärem Metabolisierungssystem für die Dauer der Untersuchung im Zuführsystem (24, 40) verbleibt.

8. Expositionsvorrichtung (10) nach einem der Ansprüche 4 bis 7, **dadurch gekennzeichnet, dass** der permeable Träger (30) derart ausgebildet ist, dass er einerseits die Aufnahme des biologischen Prüfsystems (28) und andererseits eine Trennung der gasförmigen Phase, also der Expositionsatmosphäre, von der flüssigen Phase, also des Erhaltungsmediums mit extrazellulärem Metabolisierungssystem, ermöglicht.

9. Expositionsvorrichtung (10) nach einem der Ansprüche 4 bis 8, **dadurch gekennzeichnet, dass** ein Ableitungssystem (60) vorgesehen ist, über das die Expositionsatmosphäre nach einer vorgegebenen Verweilzeit innerhalb der Expositionsvorrichtung (10) aus dieser (10) ableitbar ist.

10. Expositionsvorrichtung (10) nach einem der Ansprüche 4 bis 9, **dadurch gekennzeichnet, dass** das Zuleitungs- (50, 52) und/oder Ableitungssystem (60) derart ausgebildet sind, dass das gasförmige Medium kontrolliert mittels eines Unterdruck-Systems oder eines Überdruck-Systems durch die Expositionsvorrichtung (10) leitbar ist.

11. Expositionsvorrichtung (10) nach einem der Ansprüche 4 bis 10, **dadurch gekennzeichnet, dass** ein Abführsystem (44) vorgesehen ist, über das das Erhaltungsmedium mit extrazellulärem Metabolisierungssystem nach einer vorgegebenen Verweilzeit innerhalb der Expositionsvorrichtung (10) aus dieser (10) abführbar ist.

12. Expositionsvorrichtung (10) nach Anspruch 11, **dadurch gekennzeichnet, dass** das Zuführ- (24, 40) und Abführsystem (44) derart ausgebildet ist, dass das darin enthaltene Erhaltungsmedium mit extrazellulärem Metabolisierungssystem in einem kontinuierlichen oder pulsierenden Fluss durch das Zuführ- (24, 40) und Abführsystem (44) geführt wird, wobei zu diesem Zweck vorzugsweise wenigstens eine Pumpe vorgesehen ist.

13. Expositionsvorrichtung (10) nach einem der Ansprüche 4 bis 12, **dadurch gekennzeichnet, dass** wenigstens eine Heizvorrichtung (20, 48) vorgesehen ist, durch die die Expositionsvorrichtung (10) ganz oder teilweise temperierbar ist.

14. Expositionsvorrichtung (10) nach einem der Ansprüche 4 bis 13, **dadurch gekennzeichnet, dass** das biologische Prüfsystem (28) entweder eine Eukaryonten-Kultur, insbesondere Zelllinien, Primärzellisolate, Gewebeschnitte, rekonstruierte Gewebe wie Kokulturen, oder genetisch veränderte Zellen, oder eine Prokaryonten-Kultur ist.

15. Expositionsvorrichtung (10) nach einem der Ansprüche 4 bis 14, **dadurch gekennzeichnet, dass** diese (10) mit einer zellbasierten Sensorik kombiniert ist.

16. Verwendung des Verfahrens nach einem der Ansprüche 1 bis 3 oder der Expositionsvorrichtung (10) nach einem der Ansprüche 4 bis 15 zur Exposition wenigstens eines biologischen Prüfsystems (28) in Zigarettenrauch oder in Abgasen, vorzugsweise in Automobilabgasen oder in Industrieabgasen.

17. Verwendung des Verfahrens nach einem der Ansprüche 1 bis 3 oder der Expositionsvorrichtung (10) nach einem der Ansprüche 4 bis 15 zur Untersuchung von Umweltatmosphären, Arbeitsplatzatmosphären oder Innenraumatmosphären.

18. Verwendung des Verfahrens nach einem der Ansprüche 1 bis 3 oder der Expositionsvorrichtung (10) nach einem der Ansprüche 4 bis 15 zur Untersuchung der Wirkung eines gasförmigen Mediums auf ein biologisches Prüfsystem (28) auf dem Gebiet Pharmaentwicklung, Produktionsüberwachung oder Medizintechnik.

19. Verwendung des Verfahrens nach einem der Ansprüche 1 bis 3 oder der Expositionsvorrichtung (10) nach einem der Ansprüche 4 bis 15 zur Untersuchung der Wirkung eines gasförmigen Mediums auf ein biologisches Prüfsystem (28), nämlich zur Untersuchung toxikologischer Effekte, gentoxischer Effekte, immunmodulatorischer oder immuntoxischer Effekte.

## Claims

1. A method for analysing the effect of a gaseous medium on a biological test system (28) using an extracellular metabolisation system, comprising the following method steps
- cultivating a biological test system (28) on a permeable carrier (30)
- conducting the gaseous medium across the surface of the biological test system (28) to form an exposure atmosphere above the biological test system (28)
- adding the extracellular metabolisation system to a sustainment medium
- positioning the sustainment medium with extracellular metabolisation system below the permeable carrier (30) with contact to the permeable carrier (30) such that the sustainment medium with extracellular metabolisation system only penetrates through the permeable carrier (30), but the biological test system (28) is not flooded by the sustainment medium with extracellular metabolisation system.

2. The method according to Claim 1, **characterised by** the following further method step:
- regulating the penetration of the sustainment medium with added extracellular metabolisation system trough the permeable carrier (30) by specifying a specific pressure which acts on the sustainment medium with extracellular metabolisation system.

3. The method according to one of claims 1 to 2, **characterised by** the following further method step:
- regulating the evaporation rate above the culture (28) by specifying a specific temperature in the immediate area surrounding the biological test system (28) and/or specifying a specific flow rate of the gaseous medium flowing across the surface of the biological test system (28).

4. An exposure device (10) for carrying out the method according to one of Claims 1 to 3, comprising
- at least one permeable carrier (30) on which a biological test system (28) is cultivated,
- at least one feed system (52) via which a gaseous medium can be conducted across the surface of the biological test system (28) to form an exposure atmosphere above the biological test system (28)
- a supply system (24, 40) which contains a sustainment medium with extracellular metabolisation system and is formed such that the sustainment medium with extracellular metabolisation system is in direct contact with the underside of the permeable carrier (30), wherein
- the permeable carrier (30) is formed and arranged within the exposure device (10) such that said permeable carrier (30) physically separates the sustainment medium with extracellular metabolisation system from the exposure atmosphere, wherein
- the sustainment medium with extracellular metabolisation system can be pressed in a defined manner through only the permeable carrier (30) by means of a specified pressure, namely such that the sustainment medium with extracellular metabolisation system penetrates through the permeable carrier (30), but the biological test system (28) is not flooded by the sustainment medium with extracellular metabolisation system.

5. The exposure device (10) according to Claim 4, **characterised in that** the supply system (24, 40) is sealed off from the feed system (50, 52) and the exposure atmosphere and the sustainment medium including extracellular metabolisation system can come into contact with the exposure atmosphere under specified conditions only through the permeable carrier (30).

6. The exposure device (10) according to Claim 4, **characterised in that** the specified pressure can be adjusted hydrostatically or by means of at least one pump.

7. The exposure device (10) according to one of Claims 4 to 6, **characterised in that** the supply system (24, 40) is formed such that the sustainment medium with extracellular metabolisation system contained therein remains in the supply system (24, 40) for the duration of the analysis.

8. The exposure device (10) according to one of Claims 4 to 7, **characterised in that** the permeable carrier (30) is formed such that, on the one hand, it enables the accommodation of the biological test system (28) and, on the other hand, enables a separation of the gaseous phase, i.e. the exposure atmosphere, from the liquid phase, i.e. of the sustainment medium with extracellular metabolisation system.

9. The exposure device (10) according to one of Claims 4 to 8, **characterised in that** a lead-off system (60) is provided via which the exposure atmosphere can be led off from said exposure device (10) after a specified time spent within the exposure device (10).

10. The exposure device (10) according to one of Claims 4 to 9, **characterised in that** the feed (50, 52) and/or lead-off system(s) (60) is/are formed such that the gaseous medium can be conducted through the exposure device (10) in a controlled manner by means of an underpressure system or an overpressure system.

11. The exposure device (10) according to one of Claims 4 to 10, **characterised in that** a removal system (44) is provided via which the sustainment medium with extracellular metabolisation system can be removed from said exposure device (10) after a specified time spent within the exposure device (10).

12. The exposure device (10) according to Claim 11, **characterised in that** the supply (24, 40) and removal system(s) (44) is/are formed such that the sustainment medium with extracellular metabolisation system contained therein is guided in a continuous or pulsing flow through the supply (24, 40) and removal system(s) (44), wherein to this end at least one pump is preferably provided.

13. The exposure device (10) according to one of Claims 4 to 12, **characterised in that** at least one heating device (20, 48) is provided by means of which the temperature of the exposure device (10) can be entirely or partially maintained.

14. The exposure device (10) according to one of Claims 4 to 13, **characterised in that** the biological test system (28) is either a eukaryote culture, in particular cell lines, primary cells isolates, tissue sections, reconstructed tissues such as cocultures, or genetically modified cells, or a prokaryote culture.

15. The exposure device (10) according to one of Claims 4 to 14, **characterised in that** it (10) is combined with a cell-based sensor system.

16. A use of the method according to one of Claims 1 to 3 or of the exposure device (10) according to one of Claims 4 to 15 to expose at least one biological test system (28) in cigarette smoke or in exhaust gases, preferably in automotive exhaust gases or in industrial waste gases.

17. The use of the method according to one of Claims 1 to 3 or of the exposure device (10) according to one of Claims 4 to 15 to analyse environmental atmospheres, workplace atmospheres or interior room atmospheres.

18. The use of the method according to one of Claims 1 to 3 or of the exposure device (10) according to one of Claims 4 to 15 to analyse the effect of a gaseous medium on a biological test system (28) in the field of pharmaceutical development, production monitoring or medical technology.

19. The use of the method according to one of Claims 1 to 3 or of the exposure device (10) according to one of Claims 4 to 15 to analyse the effect of a gaseous medium on a biological test system (28), namely to analyse toxicological effects, genotoxic effects, immunomodulatory or immunotoxic effects.

## Revendications

1. Procédé servant à examiner l'effet d'un milieu gazeux sur un système de contrôle biologique (28) en utilisant un système de métabolisation extracellulaire comprenant les étapes de procédé suivantes:
- mettre en culture un système de contrôle biologique (28) sur un support perméable (30),
- faire passer le milieu gazeux sur la surface du système de contrôle biologique (28), afin de former une atmosphère d'exposition au-dessus du système de contrôle biologique (28),
- ajouter le système de métabolisation extracellulaire à un milieu de conservation,
- positionner le milieu de conservation additionné du système de métabolisation extracellulaire au-dessous du support perméable (30) et en contact avec celui-ci, de manière que le milieu de conservation additionné du système de métabolisation extracellulaire traverse uniquement le support perméable (30) et que le système de contrôle biologique (28) n'est pas inondé par le milieu de conservation additionné du système de métabolisation extracellulaire.

2. Procédé suivant la revendication 1, **caractérisé par** les autres étapes de procédé suivantes :
- réguler le passage par le support perméable (30) du milieu de conservation additionné du système de métabolisation extracellulaire en prédéfinissant une pression déterminée qui agit sur le milieu de conservation additionné du système de métabolisation extracellulaire.

3. Procédé suivant une des revendication 1 à 2, **caractérisé par** l'étape de procédé suivante :
- Réguler le taux d'évaporation au-dessus de la culture (28) en prédéfinissant une température déterminée dans l'environnement immédiat du système de contrôle biologique (28) et/ou en prédéfinissant une vitesse d'écoulement déterminée du milieu gazeux passant sur la surface du système de contrôle biologique (28).

4. Dispositif d'exposition (10) servant à exécuter le procédé suivant une des revendications 1 à 3, comprenant
- au moins un support perméable (30), sur lequel est mis en culture un système de contrôle biologique (28),
- au moins un système d'alimentation (52), par l'intermédiaire duquel un milieu gazeux peut être dirigé de manière à passer sur la surface du système de contrôle biologique (28), afin de former une atmosphère d'exposition au-dessus du système de contrôle biologique (28).
- un système d'amenée (24,40) qui contient un milieu de conservation additionné d'un système de métabolisation extracellulaire et est conçu de manière que le milieu de conservation additionné du système de métabolisation extracellulaire se trouve en contact direct avec la face inférieure du support perméable (30),
- le support perméable (30) étant conçu et disposé à l'intérieur du dispositif d'exposition (10) de manière que celui-ci (30) sépare physiquement le milieu de conservation additionné du système de métabolisation extracellulaire de l'atmosphère d'exposition,
- le milieu de conservation additionné du système de métabolisation extracellulaire pouvant être poussé, de manière définie, uniquement à travers le support perméable (30) au moyen d'une pression prédéfinie et ceci de manière que le milieu de conservation, additionné du système de métabolisation extracellulaire, traverse le support perméable (30) sans pour autant que le système de contrôle biologique (28) soit inondé par le système de métabolisation extracellulaire.

5. Disposition d'exposition (10) suivant la revendication 4, **caractérisé en ce que** le système d'amenée (24, 40) est étanchéifié par rapport au système d'alimentation (50, 52) et par rapport à l'atmosphère d'exposition et **en ce que** c'est uniquement à travers le support perméable (30) que le milieu de conservation additionné du système de métabolisation extracellulaire peut entrer en contact avec l'atmosphère d'exposition dans des conditions prédéfinies.

6. Dispositif d'exposition (10) suivant la revendication 4, **caractérisé en ce que** la pression prédéfinie est hydrostatique ou réglable au moyen d'une pompe au moins.

7. Dispositif d'exposition (10) suivant une des revendications 4 à 6, **caractérisé en ce que** le système d'amenée (24, 40) est conçu de manière que le milieu de conservation additionné du système de métabolisation extracellulaire, qu'il contient, demeure dans le système d'amenée (24, 40) pendant la durée de l'examen.

8. Dispositif d'exposition (10) suivant une des revendications 4 à 7, **caractérisé en ce que** le support perméable (30) est conçu de manière qu'il permet, d'une part, la réception du système de contrôle biologique (28) et, d'autre part, une séparation de la phase gazeuse, c'est-à-dire de l'atmosphère d'exposition, de la phase liquide, c'est-à-dire du milieu de conservation additionné du système de métabolisation extracellulaire.

9. Dispositif d'exposition (10) suivant une des revendications 4 à 8, **caractérisé en ce qu'**un système d'évacuation (60) est prévu, par l'intermédiaire duquel l'atmosphère d'exposition peut être évacuée du dispositif d'exposition (10), après une certaine durée de séjour prédéfinie à l'intérieur de celui-ci.

10. Dispositif d'exposition (10) suivant une des revendications 4 à 9, **caractérisé en ce que** le système d'alimentation (50, 52) et/ou le système d'évacuation (60) est conçu de manière que le dispositif d'exposition (10) peut être traversé par le milieu gazeux de manière contrôlée au moyen d'un système de sous-pression ou d'un système de surpression.

11. Dispositif d'exposition (10) suivant une des revendications 4 à 10, **caractérisé en ce qu'**un système d'évacuation (44) est prévu, par l'intermédiaire duquel le milieu de conservation additionné du système de métabolisation extracellulaire peut être évacué du dispositif d'exposition (10) après une durée de séjour prédéfinie à l'intérieur de celui-ci.

12. Dispositif d'exposition (10) suivant la revendication 11, **caractérisé en ce que** le système d'amenée (24, 40) et d'évacuation (44) est conçu de manière que le milieu de conservation additionné du système de métabolisation extracellulaire, qu'il contient, est dirigé en forme de flux continu ou pulsant à travers le système d'amenée (24, 40) et le système d'évacuation (44), une pompe au moins étant, de préférence, prévue à cet effet.

13. Dispositif d'exposition (10) suivant une des revendications 4 à 12, **caractérisé en ce qu'**un dispositif de chauffage (20,48) au moins est prévu, par l'intermédiaire duquel le dispositif d'exposition (10) peut être intégralement ou partiellement tempéré.

14. Dispositif d'exposition (10) suivant une des revendications 4 à 13, **caractérisé en ce que** le système de contrôle biologique (28) est soit une culture d'eucaryotes, en particulier des lignées cellulaires, des isolats de cellules premières, des coupes de tissu, des tissus reconstruits, tels que des co-cultures ou des cellules génétiquement modifiées ou une culture de procaryotes.

15. Dispositif d'exposition (10) suivant une des revendications 4 à 14, **caractérisé en ce qu'**il (10) est combiné à des biocapteurs cellulaires.

16. Utilisation du procédé suivant une des revendications 1 à 3 ou du dispositif d'exposition (10) suivant une des revendications 5 à 15 pour l'exposition d'un système de contrôle biologique (28) au moins à de la fumée de cigarettes ou à des gaz d'échappement, de préférence des gaz d'échappement de véhicules automobiles ou des gaz d'échappement industriels.

17. Utilisation du procédé suivant une des revendications 1 à 3 ou du dispositif d'exposition (10) suivant une des revendications 4 à 15 pour examiner des atmosphères de l'environnement, des atmosphères de lieux de travail ou des atmosphères d'intérieurs.

18. Utilisation du procédé suivant une des revendications 1 à 3 ou du dispositif d'exposition (10) suivant une des revendications 4 à 15 pour examiner l'effet d'un milieu gazeux sur un système de contrôle biologique (28) dans le domaine du développement pharmaceutique, de la surveillance de fabrication ou de la technique médicale.

19. Utilisation du procédé suivant une des revendications 1 à 3 ou du dispositif d'exposition (10) suivant une des revendications 4 à 15 pour l'examen de l'effet d'un milieu gazeux sur un système de contrôle biologique (28), notamment pour examiner des effets toxicologiques, des effets génotoxiques, des effets immunomodulateurs ou immunotoxiques.
